# EUROPEAN PATENT SPECIFICATION

(11) **EP 2 010 049 B1**
(45) Date of publication and mention of the grant of the patent: **11.05.2016**
(21) Application number: 07718529.6
(22) Date of filing: 01.03.2007
(51) Int. Cl.: A61B 8/06, G01R 33/48, A61B 5/00, A61B 8/00, A61B 6/00, A61B 8/08, A61B 6/03, A61B 90/00

(54) **ULTRASOUND IN MAGNETIC SPATIAL IMAGING APPARATUS**
ULTRASCHALL IN EINEM MAGNETISCHEN GERÄT ZUR RAUMDARSTELLUNG
PROCÉDÉ D'ULTRASONS DANS UN APPAREIL D'IMAGERIE SPATIALE MAGNÉTIQUE

(30) Priority: 15.03.2006 AU 2006901321
(43) Date of publication of application: 07.01.2009
(73) Proprietor: Compumedics Limited, Abbotsford, Victoria 3067 (AU)
(72) Inventor: WIDENHORN, Gerold, 88662 Überlingen (DE)
(74) Representative: Handsome I.P. Ltd
(86) International application number: PCT/AU2007/000233
(87) International publication number: WO 2007/104079

(56) References cited:
- WO-A-2007/019216
- WO-A1-00/71207
- WO-A1-95/02361
- JP-A- 6 114 055
- US-A- 5 379 770
- US-A- 5 402 789
- US-A- 5 433 206
- US-A- 5 752 515
- US-A1- 2003 195 413
- US-A1- 2004 267 111
- US-B1- 6 280 387
- PATENT ABSTRACTS OF JAPAN & JP 2005 073764 A (HITACHI MEDICAL CORP) 24 March 2005
- PATENT ABSTRACTS OF JAPAN & JP 2003 180696 A (OLYMPUS OPTICAL CO LTD) 07 July 2003

## Description

### Field of the Invention

This invention relates to methods and apparatus for measuring physiological parameters, in particular blood flow rates and electrophysiological activity, using ultrasound and spatial imaging techniques.

### Background

Developments in imaging techniques have made it possible to measure physiological parameters over differing timeframes by exploiting the physics of waveforms as they pass through heterogeneous tissues. For example, it is known in the art that the properties of the diffraction of sound waves of very high frequency (known as the Doppler effect) directed toward moving fluids, including blood moving through vessels, can be analysed to measure blood flowrate. Such techniques using sound waves (known as ultrasound technologies) have been conveniently used in applications of cardiology and, more recently, brain blood-flow. Further developments in the use of ultrasound in brain blood-flow measurements are known in the art where measurements are made using ultrasound penetrating the cranium (Transcranial Doppler, or TCD). Such techniques were disclosed by Aaslid in US patent no. 4,817,621, which described apparatus for TCD to measure blood-flow in vessels in the brain.

Other developments for making images of tissue in situ have occurred independently, using different principles of physics, such as measuring concentrations of particular atoms and molecules with varying concentrations in heterogeneous tissues by activating the atoms and molecules by strong magnetic fields and measuring the activation. Such a technique is known in the art of magnetic resonance imaging (MRI).

Both ultrasound and MRI are used to measure aspects of fluids in tissues. However, the techniques, like other imaging techniques, operate on different timescales and can be interpreted for different physiological parameters. The speed with which ultrasound waves can be generated, reflected and analysed is over periods of milliseconds. The speed at which MRI images can be generated is at least tenfold slower. Ultrasound measurements show immediate and dynamically changing flow rates of fluids such as blood. Ultrasound measurements inform little about anatomy or morphology of the tissues being penetrated. MRI measurements are static but very rich in anatomical and morphological information.

A great advantage would occur if physiological information could be derived from both ultrasound and MRI measurements of a tissue. This would be particularly advantageous for TCD blood-flow measurements of brain tissue made concurrently with MRI structural measurements. A device and method for concurrently taking ultrasound and MRI measurements, or at least in quick succession, of the same tissue could collectively provide much more information that independent, non-concurrent measurements made using either technique.

While making concurrent or quick successive measurements of physiological parameters using ultrasound and other imaging techniques like MRI would be advantageous, it has not been possible to date. A major reason is because the strong magnetic fields of MRI induce currents in electrical conducting materials. Such conducting materials are commonly used in ultrasound devices. The currents interfere with, and make it impossible to generate and receive, ultrasound waves for analysis. What is needed is ultrasound apparatus, In particular TCD apparatus, that is unaffected, or minimally affected by the magnetic fields of imaging systems such as MRI systems, to enable the concurrent or quick successive measurements of physiological parameters to enable independent and dynamic measurements of physiological processes. Similarly, methods for concurrently or quick successive measuring physiological parameters with ultrasound and MRI are needed. Such methods and apparatus would have many applications such as measuring blood flow during disease events such as stroke which is associated with abnormal blood flows in the brain.

WO9502361A1 discloses a Doppler ultrasound device to measure the flow of blood in certain locations in an arterial tree, having an MR system having a strong magnetic field and an ultrasound probe that is shielded to permit operation in the RF field of an MRI system. A triaxial lead/cable is connected to transducers on the probe, a monitor is connected to the triaxial lead/cable, the monitor having shielding, wherein the shielding allows operation of the monitor in the strong magnetic field in/near the MR system.

### Summary of the Invention

It is known in the art that ultrasound transducer probes for generating, transmitting and receiving ultrasound waves are comprised of magnetic materials including a coil for amplifying the reflected ultrasound signal received by the probe. Conventionally within the member is a crystal that vibrates to produce the ultrasound waves, the crystal being adjacent or nearly adjacent the amplifying coil needed to amplify signals for processing. However, placing such a probe within the magnetic field of magnetic imaging system such as an MRI scanner results in the induction of electrical currents in the coils by the magnetic field of the imaging system, the result being that the ultrasound probe is unusable for generating and receiving meaningful ultrasound signals. Surprisingly, the invention provides that the received ultrasound signal amplifier can be spatially separated from the ultrasound-generating crystal, outside the strong magnetic field of the imaging system, but in electrical communication with a suitable conducting connector so that the received ultrasound signal may be amplified and further processed outside the magnetic field. The result is that ultrasound signals representative of physiological processes can be measured in quick succession with the operation of the magnetic imaging system, notwithstanding the presence of the ultrasound member within the magnetic field of the magnetic imaging system.

The invention most advantageously provides apparatus and methods for measuring both ultrasound signals and magnetic imaging modalities. In one aspect the invention most advantageously provides an apparatus for making transcranial Doppler ultrasound measurements of blood flow in a subject located in an imaging device (10) having a strong magnetic field (3), including: at least one ultrasound member (4) for generating ultrasound waves within a frequency range of 1MHz to 4MHz, said member having minimal magnetic parts; electrical signal communication means (6) in communication with the at least one ultrasound member (4); electrical signal amplifying means (7) in communication with the at least one ultrasound member (4) via the electrical signal communication means (6); an imaging device (10) having a strong magnetic field (3); a magnetic field barrier (8) for magnetically shielding the signal amplifying means (7) from the strong magnetic field (3) of the imaging device (10); and an ultrasound signal processing and analysing apparatus located outside the magnetic field, and spatially separated from the electrical signal amplifying means (7), the ultrasound signal processing and analysing apparatus configured to receive electrical signals from the electrical signal amplifying means (7).

Preferably, the ultrasound probe for produce ultrasound waves in the frequency range of 2.0 MHz to 2.5 MHz. Preferably, the ultrasound member includes no electronic components. Preferably, the apparatus contains minimal ferromagnetic parts in the ultrasound member. The ultrasound member may include conducting parts comprised of carbon materials. Other embodiments include parts in the ultrasound member being comprised of substances that minimise artefacts or distortion to the. image modality image and analysis processing. The elements for acquiring and analysing ultrasound waves may be located in separate rooms from the imaging device wherein the magnetic field barrier is the wall of an enclosure. Preferably, the apparatus includes a headband for positioning at least one ultrasound member for taking ultrasound measurements. Preferably, the headband is comprised of a non-magnetic material or materials. Preferably the spatial imaging device is any one of an MRI scanner, PET scanner, or CT scanner.

According to a second aspect, there is provided a method of making transcranial Doppler ultrasound imaging measurements using ultrasound apparatus within the magnetic field (3) of a spatial imaging device (10) having a magnetic field (3) successively with making magnetic spatial images with the spatial imaging device (10), the method including the steps of: positioning at least one non-magnetic ultrasound member (4) adjacent a subject (1) for making an ultrasound measurement; establishing electrical communication with the at least one non-magnetic ultrasound member (4) with an ultrasound signal pre-amplifier (7); magnetically isolating the magnetic field of the spatial imaging device (10) from said signal pre- amplifying means (7); establishing electrical communication between the ultrasound signal pre-amplifier (7) and an ultrasound signal processing and analysing apparatus located outside the magnetic field, and spatially separated from the electrical signal amplifying means (7); operating the at least one ultrasound member (4) to produce ultrasound waves within a frequency range of 1MHz to 4MHz; operating the ultrasound signal processing and analysing apparatus to create a physiological signal of blood flow in the subject (1); interrupting the operation of the ultrasound member (4) and ultrasound signal processing and analysing apparatus; and operating the spatial imaging device (10) to record an image during said interruption. Preferably, the ultrasound waves are directed toward the head of a subject.

### Brief Description of the Figures

Figure 1 shows a diagram of the features of the invention.
Figure 2 shows an embodiment of non-magnetic ultrasound members for use in magnetic fields.
Figure 3 shows a side view of a non-magnetic ultrasound member held in place with a non-magnetic band for use in magnetic fields.
Figure 4 shows a front view of two non-magnetic ultrasound members held in place with a non-magnetic band for use in magnetic fields.

### Detailed Description of the Figures and Most Preferred Embodiments

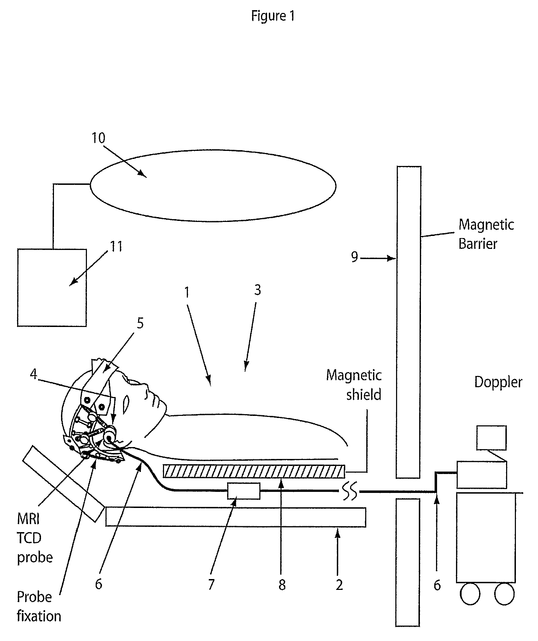

The invention is most easily understood with reference to the accompanying figures. Figure 1 shows an embodiment of the invention, including the elements necessary to acquire ultrasound signals and spatial imaging information using strong magnetic fields. It will be understood that other embodiments of the invention are possible and that the scope of the invention is not limited to the embodiments described herein. In Figure 1 is shown a subject 1 in a prone position on a table 2 within the magnetic field 3 of a spatial imaging device 10. The spatial imaging device may be any suitable spatial imaging device having a magnetic field. Preferably, the spatial imaging device is an MRI device. Other spatial imaging devices such as PET or CT devices are suitable for practising the invention. An ultrasound member 4 is engaged with a band 5 which, in turn, is engaged with the head 5 of the subject 1. Only one ultrasound member is shown in Figure 1 but it is possible that more than one member may be used in practising the invention. In electrical communication with the ultrasound member 4 is a conducting lead 6 which communicates signals to a pre-amplifier 7 that is located on the side of a magnetic field barrier or shield 8 opposite to the magnetic field 3. The magnetic field barrier 8 operates to shield the pre-amplifier 7 from electrical interference caused by induction of current in the pre-amplifier and conducting lead 6. The conducting lead 6 transverses a second magnetic field barrier 9, which shields the ultrasound-signal analysis device from interference caused by the strong magnetic field 3. It is possible to practise the invention without a second magnetic field barrier. Preferably there are two magnetic field barriers as shown in Figure 1. Preferably, the ultrasound-signal analysis device is a Doppler-Box, The arrangement of the elements herein described allows an ultrasound measurement to be made with the co-operation of the ultrasound elements within and without the magnetic field 3 at a point in time. At alternate points in time, the operation of the ultrasound equipment is paused and spatial images of the subject may be made with the spatial imaging device.

The invention is best performed when the distance between the transducer crystal of the ultrasound member 4 and the pre-amplifier is as short as possible to ensure that adequately measurable ultrasound signals can be acquired. This is achieved by placing the pre-amplifier 7 at the shielded area on the shielded side of the magnetic field shield 7 close to 1 he tissue of interest with a connector for the member. This is best achieved with ultrasound members 4 having long conducting leads 6.

The apparatus of the invention includes ultrasound members that may be spatially separate from the ultrasound signal processing and analysing apparatus, preferably including being located in separate rooms to minimise interference from the magnetic field of the magnetic imaging system, wherein the magnetic field shield is a wall of an enclosure such as a room. This is shown in Figure 1 with the magnetic shield 9. In one embodiment of the invention, using an ultrasound Doppler-Box, it is also possible to locate the ultrasound Doppler-Box close to the spatial imaging device.

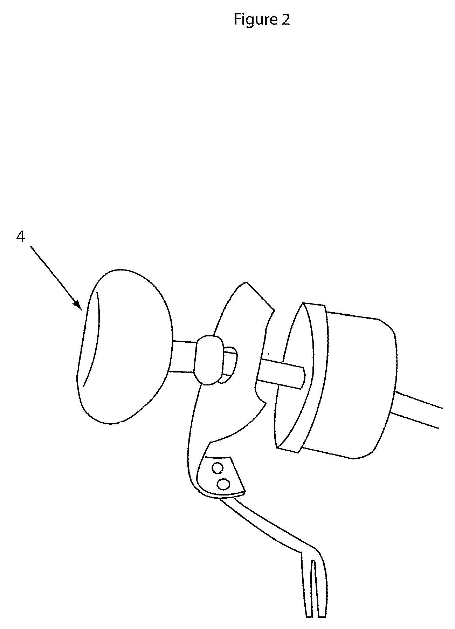

Any suitable ultrasound member may be used. Preferably the ultrasound members are probes which produce ultrasound waves within the frequency range of 1 MHz to 3 MHz. More preferably the ultrasound members are 2 MHz to 2.5 MHz ultrasound probes. Figure 2 shows an embodiment of a suitable ultrasound member 4. Preferably, the member 4 contains no electronic elements within.

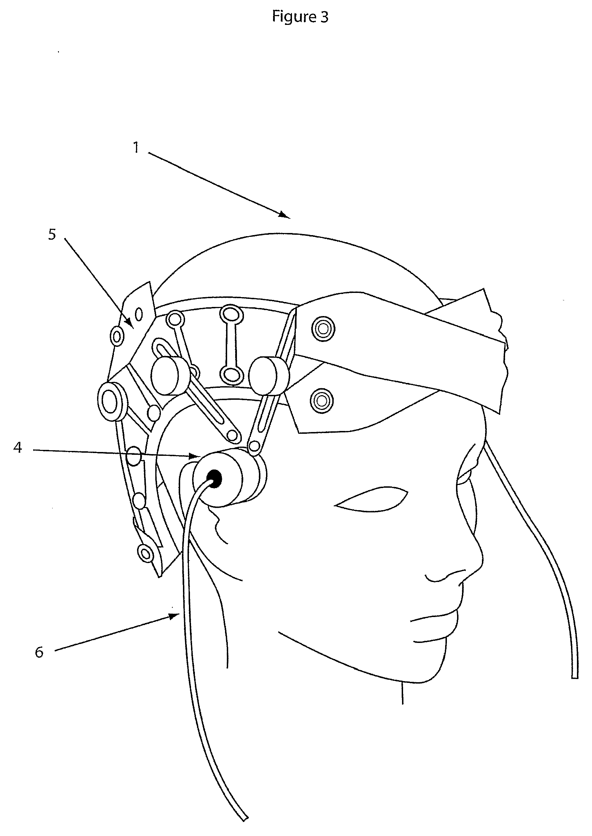

Figure 3 shows a side view of the head of a subject 1 with a headband 5 for ultrasound sonication engaged with an ultrasound member 4 in fixed in position for sonication. Preferably, all parts of the headband 5 are constructed of non-magnetic materials.

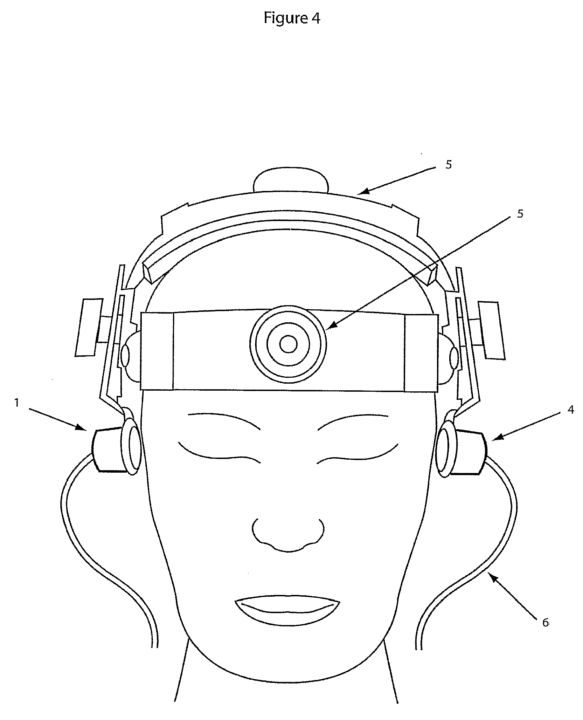

Figure 4 shows a front view of the head of a subject 1 with an alternative embodiment of the Invention with an ultrasound member 4 on each side of the head of the subject. A conducting lead 6 in conducting communication with the ultrasound member carries signals from each of the ultrasound members.

It will be understood that the invention is not limited to combining ultrasound apparatus with MRI apparatus but also includes apparatus for other spatial imaging devices including strong magnetic fields such as those implementing CT and PET.

## Claims

1. Apparatus for making transcranial Doppler ultrasound measurements of blood flow in a subject located in an imaging device (10) having a strong magnetic field (3), including:
at least one ultrasound member (4) for generating ultrasound waves within a frequency range of 1MHz to 4MHz, said at least one ultrasound member having minimal magnetic parts;
electrical signal communication means (6) in communication with the at least one ultrasound member (4);
electrical signal amplifying means (7) in communication with the at least one ultrasound member (4) via the electrical signal communication means (6);
the imaging device (10) having the strong magnetic field (3);
a magnetic field barrier (8) for magnetically shielding the electrical signal amplifying means (7) from the strong magnetic field (3) of the imaging device (10); and
an ultrasound signal processing and analysing apparatus located outside the magnetic field, and spatially separated from the electrical signal amplifying means (7), the ultrasound signal processing and analysing apparatus configured to receive electrical signals from the electrical signal amplifying means (7).

2. The apparatus of claim 1 wherein said at least one ultrasound member (4) is an ultrasound probe for
producing ultrasound waves in the frequency range of 2.0 MHz to 2.5 MHz.

3. The apparatus of any one of claims 1 to 2 wherein the at least one ultrasound member (4) includes no electronic components.

4. The apparatus of any one of claims 1 to 3 further comprising a headband (5) for positioning the at least one ultrasound member (4) for taking the ultrasound measurements.

5. The apparatus of claim 4 wherein said headband (5) is comprised of a non-magnetic material or materials.

6. The apparatus of any one of claims 1 to 5 wherein conducting parts of the at least one ultrasound member (4) are comprised of carbon materials.

7. The apparatus of any one of claims 1 to 6 wherein said imaging device (10) is any one of an MRI scanner, PET scanner, or CT scanner.

8. A method of making transcranial Doppler ultrasound imaging measurements using ultrasound apparatus within the magnetic field (3) of a spatial imaging device (10) having a magnetic field (3) successively with making magnetic spatial images with the spatial imaging device (10), the method including the steps of:
positioning at least one non-magnetic ultrasound member (4) adjacent a subject (1) for making an ultrasound measurement;
establishing electrical communication of the at least one non-magnetic ultrasound member (4) with an ultrasound signal pre-amplifier (7);
magnetically isolating the magnetic field of the spatial imaging device (10) from said signal pre- amplifying means (7);
establishing electrical communication between the ultrasound signal pre-amplifier (7) and an ultrasound signal processing and analysing apparatus located outside the magnetic field, and spatially separated from the electrical signal amplifying means (7);
operating the at least one non-magnetic ultrasound member (4) to produce ultrasound waves within a frequency range of 1MHz to 4MHz;
operating the ultrasound signal processing and analysing apparatus to create a physiological signal of blood flow in the subject (1);
interrupting the operation of the at least one non-magnetic ultrasound member (4) and the ultrasound signal processing and analysing apparatus; and
operating the spatial imaging device (10) to record an image during said interruption.

9. The method of claim 8 wherein the ultrasound wave frequency range is 2.0 MHz to 2.5 MHz.

10. The method of claim 8 or claim 9 wherein the ultrasound waves are directed toward the head of the subject.

## Patentansprüche

1. Vorrichtung zur Durchführung von transkranialen Doppler-Ultraschall-Messungen des Blutstroms in einem Subjekt, welches in einer Bildwiedergabevorrichtung (10) angeordnet ist, die ein starkes Magnetfeld (3) hat, umfassend:
wenigstens ein Ultraschallelement (4) zur Erzeugung von Ultraschallwellen innerhalb eines Frequenzbereichs von 1 MHz bis 4 MHz, wobei dieses wenigstens eine Ultraschallelement minimale magnetische Teile aufweist;
elektrische Signalübermittlungsmittel (6) in Verbindung stehend mit dem wenigstens einen Ultraschallelement (4);
elektrische Signalverstärkungsmittel (7) in Verbindung stehend mit dem wenigstens einen Ultraschallelement (4) über die elektrischen Signalübermittlungsmittel (6);
wobei die Bildwiedergabevorrichtung (10) das starke Magnetfeld (3) aufweist;
eine Magnetfeldbarriere (8) zur magnetischen Abschirmung der elektrischen Signalverstärkungsmittel (7) gegen das starke magnetische Feld (3) der Bildwiedergabevorrichtung (10); und
eine Ultraschallsignalverarbeitungs- und -analysevorrichtung, angeordnet außerhalb des Magnetfelds und räumlich getrennt von den elektrischen Signalverstärkungsmitteln (7), wobei die Ultraschallsignalverarbeitungs- und -analysevorrichtung so konfiguriert ist, dass sie elektrische Signale von den elektrischen Signalverstärkungsmitteln (7) empfängt.

2. Vorrichtung nach Anspruch 1, bei der das wenigstens eine Ultraschallelement (4) ein Ultraschallmessgeber ist zur Erzeugung von Ultraschallwellen in dem Frequenzbereich von 2,0 bis 2,5 MHz.

3. Vorrichtung nach einem der Ansprüche 1 oder 2" bei der das wenigstens eine Ultraschallelement (4) keine elektronischen Komponenten umfasst.

4. Vorrichtung nach einem der Ansprüche 1 bis 3, weiterhin umfassend ein Kopfband (5) für die Positionierung des wenigstens einen Ultraschallelements (4) zur Aufnahme der Ultraschallmessungen.

5. Vorrichtung nach Anspruch 4, bei der das Kopfband (5) aus einem nicht-magnetischen Material oder nicht-magnetischen Materialien besteht.

6. Vorrichtung nach einem der Ansprüche 1 bis 5, bei der leitende Teile des wenigstens einen Ultraschallelements (4) aus Karbonmaterialien bestehen.

7. Vorrichtung nach einem der Ansprüche 1 bis 6, bei der die genannte Bildwiedergabevorrichtung (10) eine solche ist ausgewählt aus einem MRI-Scanner, PET-Scanner oder CT-Scanner.

8. Verfahren zur Durchführung von transkranialen Doppler-Ultraschallbild-Messungen unter Verwendung einer Ultraschallvorrichtung innerhalb des Magnetfelds (3) einer dreidimensionalen Bildwiedergabevorrichtung (10), welche eine Magnetfeld (3) aufweist, aufeinanderfolgend mit der Erzeugung von magnetischen dreidimensionalen Bildern mit der dreidimensionalen Bildwiedergabevorrichtung (10), wobei das Verfahren die Schritte umfasst:
Positionieren wenigstens eines nicht-magnetischen Ultraschallelements (4) benachbart zu einem Subjekt (1) zur Durchführung einer Ultraschallmessung;
Herstellen einer elektrischen Verbindung zwischen dem wenigstens einen nicht-magnetischen Ultraschallelement (4) und einem Ultraschallsignalvorverstärker (7); magnetisches Isolieren des Magnetfelds der dreidimensionalen Bildwiedergabevorrichtung (10) von diesen Signalvorverstärkermitteln (7);
Herstellen einer elektrischen Verbindung zwischen dem Ultraschallsignalvorverstärker (7) und einer Ultraschallsignalverarbeitungs- und -analysevorrichtung, angeordnet außerhalb des Magnetfelds und räumlich beabstandet von den elektrischen Signalverstärkungsmitteln (7);
Betätigen des wenigstens einen nicht-magnetischen Ultraschallelements (4) zur Erzeugung von Ultraschallwellen in einem Frequenzbereich von 1 MHz bis 4 MHz;
Betätigen der Ultraschallsignalverarbeitungs- und -analysevorrichtung zur Erzeugung eines physiologischen Signals des Blutstroms in dem Subjekt (1);
Unterbrechen der Betätigung des wenigstens einen nicht-magnetischen Ultraschallelements (4) und der Ultraschallsignalverarbeitungs- und -analysevorrichtung und
Handhaben der dreidimensionalen Bildwiedergabevorrichtung (10) zur Aufzeichnung eines Bildes während dieser Unterbrechung.

9. Verfahren nach Anspruch 8, bei dem der Ultraschallfrequenzbereich 2,0 MHz bis 2,5 MHz ist.

10. Verfahren nach Anspruch 8 oder 9, bei dem die Ultraschallwellen in Richtung auf den Kopf des Subjekts gerichtet sind.

## Revendications

1. Appareil pour réaliser des mesures par ultrasons Doppler transcrânien de débit sanguin dans un sujet localisé dans un dispositif d'imagerie (10) ayant un fort champ magnétique (3), comprenant :
au moins un élément à ultrasons (4) pour générer des ondes ultrasonores à l'intérieur d'une plage de fréquences de 1 MHz à 4 MHz, ledit au moins un élément à ultrasons ayant le minimum de parties magnétiques ;
un moyen de communication de signal électrique (6) en communication avec l'au moins un élément à ultrasons (4) ;
un moyen d'amplification de signal électrique (7) en communication avec l'au moins un élément à ultrasons (4) par l'intermédiaire du moyen de communication de signal électrique (6) ;
le dispositif d'imagerie (10) ayant le fort champ magnétique (3) ;
une barrière de champ magnétique (8) pour protéger magnétiquement le moyen d'amplification de signal électrique (7) contre le fort champ magnétique (3) du dispositif d'imagerie (10) ; et
un appareil de traitement et d'analyse de signal ultrasonore localisé à l'extérieur du champ magnétique, et séparé spatialement du moyen d'amplification de signal électrique (7), l'appareil de traitement et d'analyse de signal ultrasonore étant configuré pour recevoir des signaux électriques en provenance du moyen d'amplification de signal électrique (7).

2. Appareil selon la revendication 1, dans lequel ledit au moins un élément à ultrasons (4) est une sonde à ultrasons pour produire des ondes ultrasonores dans la plage de fréquences de 2,0 MHz à 2,5 MHz.

3. Appareil selon l'une quelconque des revendications 1 à 2, dans lequel l'au moins un élément à ultrasons (4) ne comprend aucun composant électronique.

4. Appareil selon l'une quelconque des revendications 1 à 3, comprenant en outre un bandeau de tête (5) pour positionner l'au moins un élément à ultrasons (4) pour prendre les mesures d'ultrasons.

5. Appareil selon la revendication 4, dans lequel ledit bandeau de tête (5) est composé d'un matériau ou de matériaux non magnétiques.

6. Appareil selon l'une quelconque des revendications 1 à 5, dans lequel des parties conductrices de l'au moins un élément à ultrasons (4) sont composées de matériaux de carbone.

7. Appareil selon l'une quelconque des revendications 1 à 6, dans lequel ledit dispositif d'imagerie (10) est l'un quelconque parmi un appareil d'IRM, un appareil de tomographie par émission de positrons ou un tomodensitomètre.

8. Procédé de réalisation de mesures d'imagerie par ultrasons Doppler transcrânien à l'aide d'un appareil à ultrasons à l'intérieur du champ magnétique (3) d'un dispositif d'imagerie spatiale (10) ayant un champ magnétique (3) successivement avec la réalisation d'images spatiales magnétiques avec le dispositif d'imagerie spatiale (10), le procédé comprenant les étapes consistant à :
positionner au moins un élément à ultrasons non magnétique (4) à côté d'un sujet (1) pour réaliser une mesure d'ultrasons ;
établir une communication électrique de l'au moins un élément à ultrasons non magnétique (4) avec un pré-amplificateur de signal ultrasonore (7) ;
isoler magnétiquement le champ magnétique du dispositif d'imagerie spatiale (10) dudit moyen de pré-amplification de signal (7) ;
établir une communication électrique entre le pré-amplificateur de signal ultrasonore (7) et un appareil de traitement et d'analyse de signal ultrasonore localisé à l'extérieur du champ magnétique, et séparé spatialement du moyen d'amplification de signal électrique (7) ;
actionner l'au moins un élément à ultrasons non magnétique (4) pour produire des ondes ultrasonores à l'intérieur d'une plage de fréquences de 1 MHz à 4 MHz ;
actionner l'appareil de traitement et d'analyse de signal ultrasonore pour créer un signal physiologique de débit sanguin dans le sujet (1) ;
interrompre le fonctionnement de l'au moins un élément à ultrasons non magnétique (4) et de l'appareil de traitement et d'analyse de signal ultrasonore ; et
actionner le dispositif d'imagerie spatiale (10) pour enregistrer une image durant ladite interruption.

9. Procédé selon la revendication 8, dans lequel la plage de fréquences d'ondes ultrasonores est 2,0 MHz à 2,5 MHz.

10. Procédé selon la revendication 8 ou la revendication 9, dans lequel les ondes ultrasonores sont dirigées vers la tête du sujet.
